# EUROPÄISCHE PATENTSCHRIFT

(11) **EP 0 582 038 B1**
(45) Veröffentlichungstag und Bekanntmachung des Hinweises auf die Patenterteilung: **18.06.1997**
(21) Anmeldenummer: 93104815.1
(22) Anmeldetag: 24.03.1993
(51) Int. Cl.: A61M 5/162, A61M 5/168

(54) **Schutzvorrichtung für einen Belüftungskanal eines Flüssigkeitsüberleitungsgerätes**
Protector for a ventilating duct of a liquid transfer device
Dispositif de protection pour une gaine de ventilation d'un appareil de transfert de liquide

(30) Priorität: 07.08.1992 DE 9210674 U
(43) Veröffentlichungstag der Anmeldung: 09.02.1994
(73) Patentinhaber: B. Braun Melsungen AG, D-34209 Melsungen (DE)
(72) Erfinder: Otto, Diether B., W-3508 Melsungen (DE); Maier, Hans-Otto, Dr., W-3503 Lohfelden (DE); Lesemann, Egon, W-3508 Melsungen (DE)
(74) Vertreter: Selting, Günther, Dipl.-Ing.

(56) Entgegenhaltungen:
- WO-A-80/02506
- WO-A-89/02291
- DE-A- 2 834 588
- DE-A- 3 048 203
- DE-U- 9 103 199

## Beschreibung

Die Erfindung betrifft eine Schutzvorrichtung für einen Belüftungskanal eines Flüssigkeitsüberleitungsgerätes, insbesondere bei einer Tropfkammer gemäß dem Oberbegriff des Anspruches 1.

Bei einer aus DE-GM 70 16 939 bekannten Schutzvorrichtung ist der Halter ein mit einem Seitenstutzen an der Öffnung des Belüftungskanals verbindbares rohrförmiges Steckteil, das ein Ventil und einen koaxial zu diesem angeordneten Bakterienfilter in Form einer in einen zylindrischen Abschnitt des Steckteiles gestopften Filtermasse aufweist. Nach Ingangsetzung einer Infusion/Transfusion entsteht in dem Flüssigkeitsbehälter ein Unterdruck, der zur Aufrechterhaltung des Volumenstromes bei Glasbehältern und zur Verhinderung der Kollabierung von Kunststoffbehältern ausgeglichen werden muß. Dieser Ausgleich wird über das in Richtung des Belüftungskanals öffnende Ventil eingeleitet, wobei die durch das Bakterienfilter einströmende Luft keimfrei ist. Die Absperr-Funktionsrichtung des Rückschlagventils zeigt zum Bakterienfilter, und es soll bei Unterbrechung der Infusion bzw. Stillstand des Lösungsstromes automatisch schließen, damit etwa in dem Belüftungskanal herabsteigende Flüssigkeit nicht zum Bakterienfilter gelangen kann. Die erwähnte bekannte Schutzvorrichtung funktioniert jedoch nicht immer in diesem Sinne, denn das Ventil, das aus einem gummielastischem Becherkörper mit axial geschlitztem Boden besteht, ist unzuverlässig. Dies ist darauf zurückzuführen, daß einbaubedingte Vorspannungen in dem Becherkörper ein dichtes gegenseitiges Anliegen der Lippen an der Schlitzung verhindern können, so daß immer kleine Undichtigkeiten vorhanden sind, durch die Flüssigkeit zum Bakterienfilter gelangen kann. In diesem Falle benetzt Infusionslösung das Bakterienfilter und es wird die Belüftungsfähigkeit eingeschränkt bzw. je nach Infusionslösung und Benetzungsdauer die Belüftung vollständig blockiert. Sowohl bei einem Glasbehälter als auch bei einem Kunststoffbehälter (Plasco-Container) behindert der im Behälter entstehende Unterdruck, sobald sich Druckgleichheit einstellt, das Ausströmen von Flüssigkeit aus dem Behälter. Des weiteren sind sogenannte Druckinfusionen in diesen Fällen nicht möglich. Ferner ist die Funktion dann nicht immer gegeben, wenn das Infusionssystem z.B. aus Gründen des Transports auf das Patientenbett gelegt wird. Auch ergeben sich unangemessen hohe Fertigungskosten.

Um die Nachteile des bekannten Ventils mit unbestimmtem öffnungsdruck und unsicherer Schließfunktion zu umgehen und die Belüftungsfähigkeit von Flüssigkeitsüberleitungsgeräten zu verbessern, sind Klappenventile im Einsatz, die im Bedarfsfalle von Hand betätigt werden müssen. Unter der Voraussetzung vorschriftsmäßiger Bedienung funktionieren sie bei Normal- und Druckinfusionen. Bei niedrigen Tropfenraten (ca. 10 bis 15 Tropfen/Minute) kann es jedoch in den Fällen, in denen die Kunststoffbehälterwandungen zu instabil sind, zu Belüftungsfehlfunktionen kommen, da aufgrund der langsamen Tropfrate zu viel Zeit vergeht, bis der erforderliche Unterdruck für die Belüftungsfunktion entsteht, wodurch wiederum das Formbeharrungsvermögen des Kunststoffes angegriffen wird, die innere Gefügespannung mit "t" abnimmt, der Kunststoff kollabiert und Flüssigkeit im Belüftungskanal herabsteigt. Sie benetzt das Bakterienfilter und - wie vorher beschrieben- kann die Funktion des Flüssigkeitsüberleitungsgerätes auch dann noch gestört sein, wenn anschließend die Tropfrate höher eingestellt wird. Das manuelle Verschließen der Klappe bei Normal-Infusionen, z.B. bei Infusionsunterbrechungen, Einstellen des Flüssigkeitsspiegels in der Tropfkammer und dergleichen, sowie bei Druckinfusionen, ist für das Personal eine Belastung, weil es ständige Anwesenheit und Aufmerksamkeit während der Infusion verlangt.

Darüberhinaus ist aus der DE-A-3 048 203 ein als beweglich aufgekängte Klappe ausgebildetes Rückschlagventil bekannt, bei dem die Klappe aus einem federelastischen Material, wie z.B. Metall besteht.

Aufgrund dieser Materialwahl ist ein Ansprechen bei geringsten Druckunterschieden nicht gewährleistet.

Eine weitere Schutzvorrichtung für einen Belüftungskanal ist in WO-A 8002506 offenbart. Diese Schutzvorrichtung weist die im Oberbegriff des Anspruches 1 angeführten Merkmale auf. Die flexible Dichtungsmembran ist dabei als geschlossene Scheibe gestaltet, deren Rand ringsum an dem Halter eingespannt ist und deren Zentrum an einen ringförmigen Sitz gezogen wird, der in bezug auf die Ebene der Randeinspannung der Dichtmembran vorspringt. Der ringförmige Sitz ist von einem Ringraum umgeben, der über einen Durchlaß mit der das Filter durchströmenden Außenluft in Verbindung steht. Ein quergerichteter Kanal in dem Halter stellt eine Verbindung her zwischen der äußeren Mündung des ringförmigen Sitzes und dem Belüftungskanal der Tropfkammer. Außenluft gelangt durch das Filter, den Durchlaß und den Ringraum gegen die Innenfläche der Dichtmembran, so daß sie gegen ihre Vorspannung vom ringförmigen Sitz abgehoben wird und einen Durchgang für die zum Belüftungskanal strömende Luft freigibt. Die Dichtmembran unterliegt durch die Überspannung des vorspringenden ringförmigen Sitzes permanenter Dehnungsverformung, die im Laufe der Zeit die ursprüngliche Elastizität der Dichtmembran verringert. Dies führt bei längerer Lagerzeit dazu, daß die Rückstellfähigkeit der Dichtmembran erlahmt und die Schließwirkung unsicher wird. Bei der Einrichtung der Schutzvorrichtung wird die Anlagekraft der Dichtmembran gegen den ringförmigen Sitz einem gewünschten, für die Funktion des Flüssigkeitsüberleitungsgerätes günstigen, Öffnungsdruck angepaßt. Die nachlassende Spannkraft der Dichtmembran verändert ihre Ansprechcharakteristik und der Durchlaß wird bei zunehmend geringer werdenden Drücken geöffnet. Es besteht die Gefahr, daß bei Unterbrechung der Infusion bzw. Stillstand des Lösungsstromes im Belüftungskanal herabsteigende Flüssigkeit zum Bakterienfilter gelangt und dieses durch Benetzung zusetzt, wodurch die Belüftung ungünstigenfalls vollständig blockiert wird. Ein Wiederingangsetzen des Infusionsstromes mit derselben Vorrichtung ist nicht möglich. Sie muß kosten- und zeitaufwendig ausgetauscht werden.

Der Erfindung liegt die Aufgabe zugrunde, die Schutzvorichtung gemäß dem Oberbegriff des Anspruches 1 so zu verbessern, daß das Ventil in allen Anwendungsfällen eines Flüssigkeitsüberleitungsgerätes mit vorbestimmtem Öffnungsdruck und sicherer Schließfunktion arbeitet, so daß eine ausreichende Belüftung des Flüssigkeitsbehälters bei sicherem Ablauf der Flüssigkeitsüberleitung gewährleistet ist.

Diese Aufgabe wird erfindungsgemäß durch die Merkmale des kennzeichnenden Teils des Anspruches 1 gelöst.

Eine solche Schutzvorrichtung, bei der die Absperr-Funktionsrichtung der Dichtmembran zum Bakterienfilter zeigt, arbeitet bei jeder Art von Flüssigkeitsübertragung sehr zuverlässig, weil bei Unterbrechung der Flüssigkeitsüberleitung bzw. Stillstand des Flüssigkeitsstromes nach Druckausgleich in dem Flüssigkeitsbehälter die vorgespannte Dichtmembran sofort automatisch dicht schließt, indem sich der Rand der Dichtmembran gegen den ringförmigen Sitz anlegt.

Die erfindungsgemäße Anordnung der Dichtmembran hat zudem den Vorteil der Lageunabhängigkeit, d.h. in kritischen Lagen des Flüssigkeitsüberleitungssystems, z.B. beim Patiententransport, wenn der Flüssigkeitsbehälter mit angeschlossenem Überleitungsgerät auf dem Bett liegend mitgeführt wird, ist das Ventil aufgrund der Vorspannung der Dichtmembran immer dicht. Das Bakterienfilter wird zuverlässig von Flüssigkeit freigehalten und seine Luftdurchlässigkeit bleibt uneingeschränkt erhalten. Der nach Ingangsetzen der Flüssigkeitsüberleitung im Flüssigkeitsbehälter entstehende Unterdruck wird durch die intakte Belüftung zuverlässig ausgeglichen und ein kontinuierlicher Flüssigkeitsstrom ohne die Gefahr des Kollabierens von Kunststoffbehältern ist gewährleistet.

Bei der im Anspruch 1 angegebenen ersten Variante der Erfindung ist die Dichtmembran nur über einen Teil ihres äußeren Umfangs haftend und/oder klemmend befestigt. Eine örtliche Einspannung der als geschlossene Scheibe gestalteten Dichtmembran läßt sich z.B. mit Hilfe eines axialen exzentrischen Zapfens oder von zwei Klemmteilen erreichen, die auf der Kreislinie des ringförmigen Sitzes gegen die Dichtmembran drücken. Dabei ist zweckmäßigerweise der ringförmige Sitz wie in Anspruch 6 angegeben schräg gerichtet.

Der ringförmige Sitz kann am inneren Ende des Stopfens ausgebildet sein. Der axial durchbrochene Stopfen, der als Kunststoffspritzling gefertigt sein kann, gestattet eine gedrungene Form der Schutzvorrichtung, so daß bei Ansetzen des Stopfens an den seitlichen Stutzen des Belüftungskanals einer Tropfkammer der Stopfen in dem Stutzen im wesentlichen versenkt eingelassen sein kann und keinen störenden Überstand bildet.

Bei der im Anspruch 1 angegebenen zweiten Variante der Erfindung ist die Dichtmembran über einen auslenkbaren Radialabschnitt mit einem äußeren Ring einstückig verbunden, der als umfangsmäßiges Randbefestigungselement dient. Die Stirnfläche eines den ringförmigen Sitz umgebenden ringförmigen Teiles und eine gegenüberliegende Gegenfläche können zu der die Längsachse des Stopfens rechtwinklig kreuzenden Ebene unter einem Winkel größer 0° und kleiner 90° versenkt konisch verlaufen. Die Fixierschrägen sind zur Erzielung der Vorspannung an der Dichtmembran günstig.

In vorteilhafter Ausgestaltung ist der ringförmige Sitz an einem Ende eines Einsatzes ausgebildet, der als in einen zentralen Hohlraum des Stopfens eingepaßter, axial durchbrochener Formkörper gestaltet ist und dessen anderes Ende ein axiales Klemmorgan zur Anpressung des Bakterienfilters gegen eine Anlageschulter des Stopfens trägt. Der Einsatz ist vorzugsweise als Kunststoffspritzling hergestellt und paßt dicht in den Hohlraum des Stopfens. Sein Zweck besteht für eine Ausführung der Erfindung darin, den ringförmigen Sitz für die Dichtmembran zu bilden, zur Befestigung der Dichtmembran beizutragen und ein inneres Gegenelement für die Befestigung des Bakterienfilters an der Anlageschulter darzustellen. Dabei ist es wichtig, den Luftraum hinter dem Bakterienfilter so groß wie möglich zu machen, um den Filterwiderstand zu reduzieren. Bei Verwendung eines Einsatzes ist dieser entsprechend ausgespart. Bei Fehlen eines Einsatzes kann dies durch entsprechende Vertiefung des Stopfenkörpers im Bereich des Bakterienfilters geschehen. Auch der Luftraum hinter der Dichtmembran soll für eine gute Ventilöffnungsfunktion so groß wie möglich sein. Zu diesem Zweck kann ebenfalls der Stopfen selbst oder der Einsatz entsprechend ausgespart sein. Je größer der Luftraum hinter der Dichtmembran ist, um so geringer ist der erforderliche Ventilöffnungsdruck.

Die Dichtmembran kann aus Naturkautschuk, thermoplastischem Elastomer oder einem anderen geeigneten Kunststoff-(Folien)material bestehen. Vorzugsweise ist die Dichtmembran silikoniert. Die Ventilfunktion der Dichtmembran ist steuerbar über die Scheibendicke, Shore-Härte des Materials, Ausführung der Kante des ringförmigen Sitzes und der Vorspannung. Hierdurch ist die Möglichkeit gegeben, den Öffnungs- und Schließdruck funktionsentsprechend zu beeinflussen.

Zur Befestigung des Bakterienfilters an dem Stopfen kann dieser von einem Ring oder Zapfen des Einsatzes gegen eine Schulter an dem Stopfen gedrückt werden oder es wird ein Bördelrand des Stopfens gegen die Außenseite des Randes des auf einer Anlageschulter ruhenden Bakterienfilters angepreßt.

Die Axialdurchbrechung des Stopfens und/oder des Einsatzes kann aus einer konzentrischen Bohrung gebildet sein.

Die Dichtmembran, der Einsatz und das Bakterienfilter können in dem Hohlraum des Stopfens vormontiert und als Funktionseinheit in den seitlichen Stutzen der Tropfkammer eingesetzt werden, so daß die Schutzvorrichtung in den Belüftungskanal integriert ist. Durch Verklebung oder Einpressen des Stopfens in dem Stutzen wird ein abgedichteter fester Sitz der Funktionseinheit erreicht.

In der Zeichnung sind Ausführungsbeispiele der Erfindung schematisch dargestellt. Es zeigt:

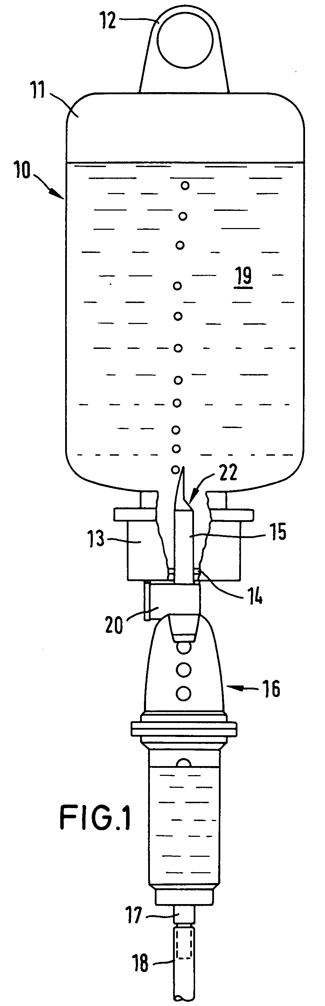

Fig. 1 eine Ansicht eines Flüssigkeitsbehälters eines Flüssigkeitsüberleitungsgerätes mit angesetzter Tropfkammer, die eine Schutzvorrichtung aufweist,
Fign. 2,2A-2C einen Längsschnitt einer ersten Ausführungsform der Erfindung sowie Stirnansichten des Stopfens und des Einsatzes,
Fign. 3 und 3A eine zweite Ausführungsform im Längsschnitt sowie eine Stirnansicht des Stopfens
Fign. 4 und 4A eine dritte Ausführungsform im Längsschnitt sowie eine Stirnansicht des Stopfens und
Fign. 5, 5A, 5B eine vierte Ausführungsform im Längsschnitt sowie einen Längsschnitt des Stopfens und Draufsicht auf die spezielle Dichtmembrananordnung.

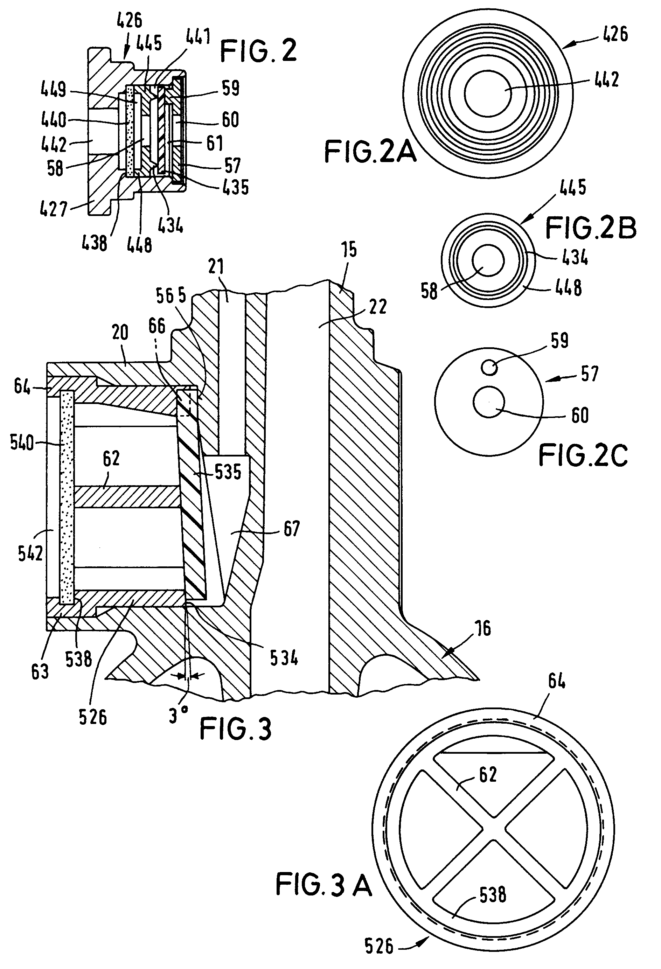

Ein Flüssigkeitsüberleitungsgerät 10 besteht im dargestellten Falle aus einem Behälter 11 aus Kunststoffmaterial, der mit Hilfe einer Öse 12 an einem Ständer so aufhängbar ist, daß sein mit einer Kappe 13 verschlossener Auslaß nach unten hängt. Die Kappe 13 enthält eine durchstechbare Membran 14, durch die ein Einstechdorn 15 einer Tropfkammer 16 hindurchgestochen ist. Am unteren Ende der Tropfkammer 16 ist an einen Stutzen 17 ein Schlauch 18 angeschlossen, der zu einem Patienten führt. Der Einstechdorn 15 der Tropfkammer besitzt zwei zueinander parallele axiale Kanäle 21, 22 (Figur 3). Der Kanal 22 mündet in den Hohlraum der Tropfkammer 16 und führt diesem tropfenweise Flüssigkeit 19 zu, während der Kanal 21 als Belüftungskanal dient, der an einer Seitenöffnung mit einem seitwärts gerichteten Stutzen 20 mündet. In den Stutzen 20 ist eine Schutzvorrichtung der in den Figuren 2 bis 5 gezeigten Ausgestaltungen eingesetzt, die zuverlässig und automatisch die Belüftung der starren und halbstarren Flüssigkeitsbehälter sowie einen zuverlässigen Abschluß des Belüftungskanals 21 nach außen, wenn die Flüssigkeitsüberleitung unterbrochen wird, gewährleistet.

Die erste Ausführungsform der Schutzvorrichtung gemäß Figuren 2, 2A-2C besteht aus einem Stopfen 426, der als Kunststoffspritzling gefertigt sein kann und einen Außenflansch 427, einen kreiszylindrischen Halsteil und einen längeren kreiszylindrischen Hauptteil aufweist. Der Stopfen 426 dient als Halter für ein kreisscheibenförmiges Bakterienfilter 440 und eine Dichtmembran 435. Die Dichtmembran 435 besteht aus Naturkautschuk, thermoplastischem Elastomer oder einem anderen geeigneten Kunststoff-(Folien)material.

Der Stopfen 426 weist im Bereich des Außenflansches 427 eine zentrale Öffnung 442 auf, die in einen kreiszylindrischen Hohlraum 441 mündet, der am inneren Ende des Stopfens 426 offen ist. In dem Hohlraum 441 ist ein Einsatz 445 untergebracht, der vorzugsweise als Kunststoffspritzling gefertigt ist. Er besitzt einen kreiszylindrischen Körper, der abdichtend in den Hohlraum 441 des Stopfens 426 paßt und der von einem zentralen Loch 58 durchsetzt ist. An der im Einbauzustand äußeren Stirnseite des Einsatzes 445 ist ein Ringrand 448 ausgebildet, der eine Vertiefung 449 mit ebener Grundfläche umschließt, und an seiner inneren Stirnseite befindet sich ein ringförmiger Sitz 434.

Der Stopfen 426 wird in den Stutzen 20 der Tropfkammer 16 eingefügt, so daß sein Außenflansch 427 einen äußeren Anschlag bildet, der die Eindringtiefe des Stopfens 426 in den Stutzen 20 so begrenzt, daß die Dichtmembran 435 genügend Platz hat, um sich in Öffnungsrichtung klappenartig zu bewegen. In geschlossenem Zustand der Dichtmembran 435 liegt sie auf dem ringförmigen Sitz 434 abdichtend auf.

In dem kreiszylindrischen Hohlraum 441 des Stopfens 426 stecken parallel zueinander vier scheibenförmige Teile, und zwar an der Außenseite ein scheibenförmiges Bakterienfilter 440, daran anschließend der Einsatz 445, die verhältnismäßig dicke Dichtmembran 435 und ein plattenförmiges Sicherungselement 57. Der Einsatz 445 drückt mit dem Ringrand 448 das Bakterienfilter 440 gegen eine Anlageschulter 438 des Stopfens 426. Zwischen dem Bakterienfilter 440 und der ebenen Grundfläche des Einsatzes 445 befindet sich die Vertiefung 449, die als Luftraum dient. Das zentrale Loch 58 in dem axial schmalen Einsatz 445 dient dem Luftdurchlaß zu der Dichtmembran 435. Der ringförmige Sitz 434 mit dreieckigem Querschnittsprofil stützt die Außenfläche der Dichtmembran 435 mit Abstand zu ihrem äußeren Rand abdichtend ab. Als Fixierelement für die Dichtmembran 435 dient ein außermittig an dem Sicherungselement 57 vorgesehener Zapfen 59, der in der Flucht des Sitzes 434 gegen die Dichtmembran 435 drückt und der in bezug auf die innere Fläche des Sicherungselementes 57 einen Luftraum 61 zur Ermöglichung der Auslenkung des freien Teils der Dichtmembran 435 bildet. Die exzentrische Klemmung der Dichtmembran 435 ist vorteilhaft, weil sie eine Funktion der Dichtmembran bei kleinem Öffnungsdruck ermöglicht. In dem plattenförmigen Sicherungselement 57 ist ein koaxiales Loch 60 zum Luftdurchlaß ausgebildet. Das Sicherungselement 57 ist in eine umfangsmäßige Aussparung am offenen Rand des Stopfens 426 eingeschweißt, eingeklebt oder eingepreßt.

Nach Ingangsetzung einer Infusion/Transfusion entsteht in dem Flüssigkeitsbehälter 11 Unterdruck. In diesem Moment wird durch die Öffnung 442 der Schutzvorrichtung Raumluft durch das Bakterienfilter 440 eingelassen, die die Dichtmembran 435 klappenartig von dem Sitz 434 wegdrückt, so daß ein Durchlaß entsteht, durch den Luft in den Belüftungskanal 21 und somit in den Flüssigkeitsbehälter 11 einströmen kann, die einen Druckausgleich herbeiführt, der für kontinuierliches Ausströmen der Flüssigkeit 19 sorgt. Bei Unterbrechung der Infusion bzw. Stillstand des Flüssigkeitsstromes schließt nach Druckausgleich die Dichtmembran 435 automatisch, so daß der im Flüssigkeitsbehälter 11 herrschende Druck erhalten bleibt und keine Flüssigkeit in den Belüftungskanal 21 eintreten kann - das Bakterienfilter 440 bleibt in jedem Falle trocken und behält seine ursprüngliche Luftdurchlässigkeit uneingeschränkt bei. Diese Funktion ist im wesentlichen bei allen Ausführungsformen die gleiche.

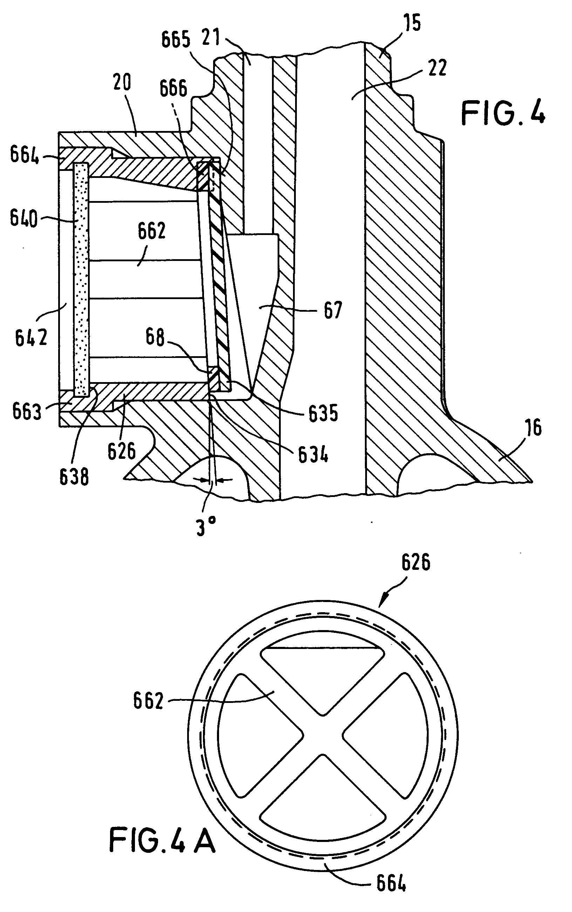

Bei den Beispielen der Figuren 3,3A und 4,4A bestehen die Stopfen 526 und 626 aus einem außen kreiszylindrischen Rohrkörper mit zentraler Bohrung 542,642, der innen mittels eines mit diesem einstückig ausgebildeten Kreuzes 62,662 versteift ist.

Der Stopfen 526 ist mit einem Außenflansch 63 in eine Ringaufnahme des Stutzens 20 der Tropfkammer 16 eingepaßt und in dieser befestigt. Durch einen Bördelrand 64 wird ein scheibenförmiges Bakterienfilter 540 gegen eine Anlageschulter 538 gepreßt. Die Anlageschulter 538 liegt in gleicher Ebene wie das in Bezug auf den Bördelrand 64 zurückgesetzte äußere Ende des Kreuzes 62, so daß das Bakterienfilter 540 auf dem Kreuz 62 abgestützt ist. Die sehr großen dreieckigen Zwischenräume zwischen den Streben des Kreuzes 62 sorgen für große durchströmende Luftmengen. An der Innenseite des Stopfens 526 bilden die Enden der Kreiswand des Stopfens 526 und des Kreuzes 62 einen ringförmigen Sitz 534 für eine geschlossenflächige Dichtmembran 535, der im Einbauzustand abwärts gegen den Belüftungskanal 21 gerichtet schräg verläuft. Die Fixierschräge des Sitzes 534 hat zur Senkrechten einen Winkel von vorzugsweise 3°. Die Dichtmembran 535 ist mit einem Teil ihres äußeren Umfanges in eine aus einem äußeren Brüstungsrand und einem Kreissegment gebildete schalenförmige Aufnahme 66 eines nach innen radial verdickten Wandungsteiles des Stopfens 526 eingesetzt und in diesem z.B. festgeklebt und/oder durch Pressung gegen eine Anschlußfläche 565 des Gehäuses eingespannt. Von der Aufnahme 66 hängt die Dichtmembran 535 durch die Abschrägung des Sitzes 534 vorgespannt nach unten. Da der Freiraum für ihre Auslenkbewegung eine Aussparung 67 am unteren Ende des Belüftungskanals 21 ist, kann der Stopfen 526 bündig mit dem Stutzen 20 abschließend in diesem versenkt werden und es entfällt die bei den anderen Ausführungsformen nötige Belassung eines scheibenförmigen Luftraumes, der die Einbaulänge des Stopfens vergrößert.

Die verhältnismäßig dicke Dichtmembran 535 bei dem Beispiel nach Figur 3 ist gemäß Figur 4 durch eine dünnere und damit flexiblere geschlossenflächige Dichtmembran 635 ersetzt. Diese ist mit einem hohlen Stopfen 626 verbunden, der bis auf breitere Streben des Kreuzes 662 gleich dem Stopfen 526 ist. Die Dichtmembran 635 ist auf der einen Seite am Rand mit einem Ring 68 aus gleichem oder ähnlichem Material versehen, der einstückig angeformt oder aufgeklebt sein kann und der den dünnen Folienkörper stabilisiert. Der Ring 68 ist mit einem kleinen Teil seines Umfanges in die entsprechend der Aufnahme 66 ausgebildete schalenförmige Aufnahme 666 des Stopfens 626 eingeklebt oder eingeschweißt und die Dichtmembran wird im Bereich der Aufnahme 666 gegen eine Anschlußfläche 665 des Gehäuses angedrückt. Sie folgt im übrigen dem entsprechend dem Sitz 534 vorgesehenen schrägen Verlauf des Sitzes 634. Während das z.B. vom Bördelrand 664 gehaltene Bakterienfilter 640 gegen das stirnseitige Ende des Kreuzes 662 und eine Anlegeschulter 638 eines Außenflansches 663 anliegt, ist die Dichtmembran 635 von dem inneren Kreuzende entfernt und die durch die Räume zwischen den Streben des Kreuzes 662 strömende Luft greift vollflächig an die Dichtmembran 635 an und bewegt sie in Öffnungsrichtung. Auch dieses Rückschlagventil arbeitet mit sehr geringen Öffnungsdrücken. Die gesamte Bauteillänge des Stopfens 626 ist auch bei diesem Beispiel in den Stutzen 20 eingelassen, so daß dieser von Überständen frei ist.

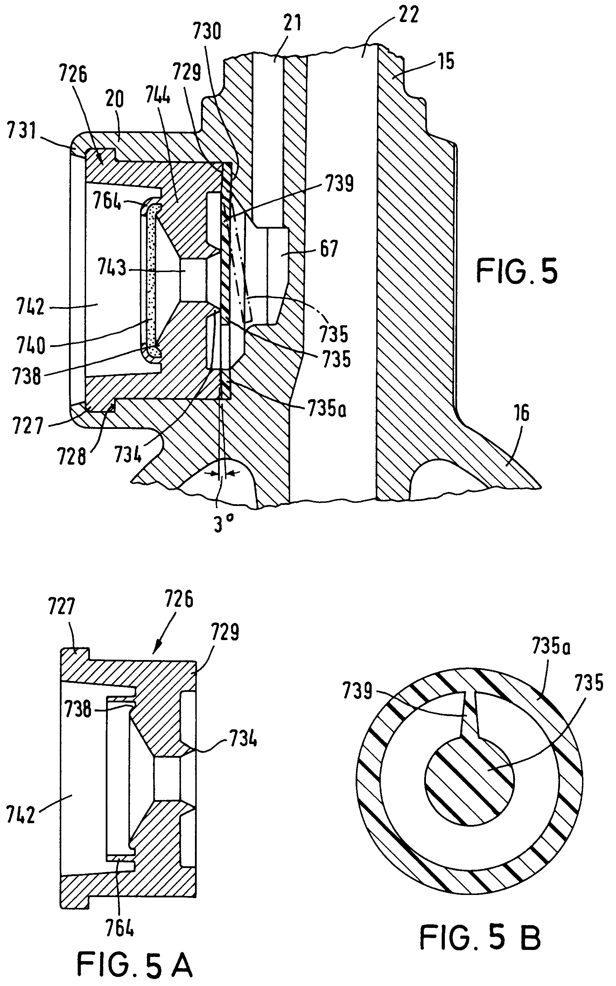

Bei dem Beispiel der Figuren 5, 5A, 5B ist eine von den vorstehend erläuterten Ausführungsformen abweichende Gestaltung des Stopfens und der Dichtmembran gewählt worden. In dem seitwärts gerichteten Stutzen 20, der über eine Seitenöffnung mit dem Belüftungskanal 21 des Einstechdornes 15 der Tropfkammer 16 in Verbindung steht, steckt ein kreiszylindrischer hohler Stopfen 726 aus Kunststoff. Der Stopfen 726 ist an seinem nach außen gewandten Ende mit einem Außenflansch 727 versehen, der gegen eine Ringauflage 728 des Stutzens 20 anliegt. Der Stopfen 726 wird durch Friktionssitz, Verklebung oder wie dargestellt durch einen Bördelrand 731 in dem Hohlraum des Stutzens 20 festgehalten. Der Stopfen 726 ist im Bereich seiner nach außen gewandten Längshälfte hohl und bildet eine zentrale Öffnung 742, die mit einer kleineren konzentrischen Öffnung 743 in Verbindung steht. Die Öffnung 743 ist in einem Bodenteil 744 des Stopfens ausgebildet, der auf der nach außen gerichteten Seite trichterförmig gestaltet ist und auf der nach innen gerichteten Seite einen konzentrischen Sitz 734 aufweist, der ringförmig gestaltet und im Querschnitt dreieckig geformt ist. Der schmale Grat des Sitzes 734 bildet das Auflager für eine Dichtmembran 735. An dem erweiterten Ende des Trichters des Bodenteils 744 ist ein Bakterienfilter 740 befestigt. Das Bakterienfilter 740 ist eine kreisförmige Schreibe mit nach einer Richtung umgebogenem Rand. Der umgebogene Rand greift in eine Rille ein, die Teil einer Anlageschulter 738 ist. Ein umlaufender Bördelrand 764 hält das Bakterienfilter 740 in seiner in den Stopfen eingelassenen Position fest. Das Bakterienfilter 740 ist zu der Öffnung 743 konzentrisch angebracht und bewirkt, daß keimfreie Luft in den Belüftungskanal 21 einströmt, wenn die Dichtmembran 735 sich in Öffnungsstellung befindet.

Die Dichtmembran 735 ist bei diesem Ausführungsbeispiel eine kleine kreisförmige Scheibe aus elastischem Material, die mit Silikonöl einer Viskosität von 350 cSt bis 30.000 cSt silikoniert ist. Sie wird konzentrisch von einem Ring 735a aus dem gleichen Material umgeben und ist mit dem Ring 735a über einen Radialsteg 739 einstückig verbunden. Der Radialsteg 739 überbrückt einen Ringraum zwischen dem Innenumfang des Ringes 735a und dem Außenumfang der Dichtmembran 735 und ermöglicht eine freie Beweglichkeit der Dichtmembran 735 quer zur Ebene des Ringes 735a. Vorzugsweise verjüngt sich der Radialsteg 739 von der Dichtmembran nach außen, so daß die Dichtmembran 735 bereits auf sehr niedrige Öffnungsdrücke anspricht. Der Ring 735a dient der Befestigung der Dichtmembran 735 an dem Stopfen 726. Zu diesem Zweck wird er zwischen der Stirnfläche eines den Sitz 734 konzentrisch umgebenden Teiles 729 des Stopfens 726 und einer Gegenfläche 730 am Grund des Stutzens 20 eingeklemmt. Die radiale Breite des Ringes 735a und der Stirnfläche des Teiles 729 sind im wesentlichen gleich. Gegebenenfalls kann zusätzlich zu der Einspannung eine Haftverbindung vorgesehen sein. Die Aussparung 67 am unteren Ende des Belüftungskanals 21 bietet Freiraum für die Auslenkbewegung der Dichtmembran 735 in Öffnungsrichtung, wie in Fig. 4 gestrichelt angedeutet ist.

Zur Erzielung guter Schließkraft der Dichtmembran 735 in Bezug auf den Sitz 734 ist die Dichtmembran 735 in Schließrichtung vorgespannt. Zu diesem Zweck verläuft die Stirnfläche des ringförmigen Teiles 729 des Stopfens 726 unter einem Winkel von vorzugsweise 3° zur senkrechten Ebene schräg, so daß eine versenkte konische Auflage für den Ring 735a gebildet ist. Die Gegenfläche 730 am Grund des Stutzens 20, gegen die der Ring 735a der Dichtmembran 735 angedrückt wird, ist unter demselben Winkel und in gleicher Orientierung abgeschrägt, während der Sitz 734 in der senkrechten Ebene liegt. Bei eingestecktem Stopfen 726 ergibt sich eine Fassung, die der Dichtmembran 735 die erwünschte gleichmäßige Vorspannung in Schließrichtung erteilt.

## Patentansprüche

1. Schutzvorrichtung für einen Belüftungskanal (21) eines Flüssigkeitsüberleitungsgerätes, insbesondere bei einer Tropfkammer (16), bestehend aus einem an eine Öffnung des Belüftungskanals (21) ansetzbaren Stopfen (426;526;626;726) mit axialem Durchlaß, der an seiner dem Belüftungskanal (21) zuzuwendenden Innenseite mit einem eine in Schließrichtung vorgespannte flexible Dichtmembran (435;535;635;735) aufweisenden Ruckschlagventil und an seiner dem Belüftungskanal (21) abzuwendenden Außenseite mit einem Bakterienfilter (440;540;640;740) versehen ist, wobei der Stopfen einen ringförmigen Sitz (434;534; 634;734) für die Dichtmembran und eine Anlage (438;538;536;738) zur Befestigung des Randes des scheibenförmig gestalteten Bakterienfilters (440;540;640;740) aufweist,
**dadurch gekennzeichnet**,
daß die Dichtmembran (435;535;635;735) nur über einen Teil ihres äußeren Umfangs haftend und/oder klemmend befestigt ist oder eine umfangsmäßige, haftende und/oder klemmende Randbefestigung aufweist, wobei im Fall der umfangsmäßigen Randbefestigung die Dichtmembran (735) über einen auslenkbaren Radialabschnitt (739) innerhalb eines sie mit radialem Abstand umgebenden, die Randbefestigung bildenden Ringes (735a) angeordnet ist.

2. Schutzvorrichtung nach Anspruch 1, dadurch gekennzeichnet, daß der Stopfen (426;526;626;726) in einen seitwärts gerichteten Stutzen (20) an dem Belüftungskanal (21) eingesetzt ist.

3. Schutzvorrichtung nach Anspruch 2, dadurch gekennzeichnet, daß der ringförmige Sitz (534;634;734) am inneren Ende des Stopfens (526;626;726) ausgebildet ist.

4. Schutzvorrichtung nach Anspruch 2, dadurch gekennzeichnet, daß der ringförmige Sitz (434) an einem Ende eines Einsatzes (445) ausgebildet ist, der als in einen zentralen Hohlraum (441) des Stopfens (426) eingepaßter, axial durchbrochener Formkörper gestaltet ist und dessen anderes Ende ein axiales Klemmorgan (448) zur Anpressung des Bakterienfilters (440) gegen eine Anlageschulter (438) des Stopfens (426) trägt.

5. Schutzvorrichtung nach einem der Ansprüche 1 bis 4, dadurch gekennzeichnet, daß der ringförmige Sitz (434;734) im Querschnitt dreieckig ist.

6. Schutzvorrichtung nach einem der Ansprüche 1 bis 3, dadurch gekennzeichnet, daß der ringförmige Sitz (534;634) ausgehend von der Befestigung der Dichtmembran (535;635) zu der die Längsachse des Stopfens (526;626) rechtwinklig kreuzenden Ebene unter einem Winkel von vorzugsweise 3° gegen den Belüftungskanal (21) schräg verläuft.

7. Schutzvorrichtung nach einem der Ansprüche 2 bis 5, dadurch gekennzeichnet, daß der Ring (735a) zwischen einer Stirnfläche eines den Sitz (734) umgebenden ringförmigen Teiles (729) des Stopfens (726) und einer Gegenfläche (730) am Grunde des Stutzens (20) eingespannt ist.

8. Schutzvorrichtung nach Anspruch 7, dadurch gekennzeichnet, daß die Stirnfläche des ringförmigen Teiles (729) und die Gegenfläche (730) zu der die Längsachse des Stopfens (726) rechtwinklig kreuzenden Ebene unter einem Winkel > 0° und < 90° versenkt konisch verlaufen.

9. Schutzvorrichtung nach einem der Ansprüche 1 bis 8, dadurch gekennzeichnet, daß ein Bördelrand (64;664;764) des Stopfens (526;626;726) das Bakterienfilter (540;640;740) gegen eine Anlageschulter (538;638;738) anpreßt.

10. Schutzvorrichtung nach einem der Ansprüche 4 bis 9, dadurch gekennzeichnet, daß die Axialdurchbrechung des Stopfens (426;526;626;726) und/oder des Einsatzes (445) aus einer konzentrischen Bohrung (442;542; 642;742) gebildet ist.

11. Schutzvorrichtung nach einem der Ansprüche 1 bis 10, dadurch gekennzeichnet, daß die Dichtmembran (435;535;635; 735) aus Naturkautschuk oder thermoplastischem Elastomermaterial gebildet ist.

12. Schutzvorrichtung nach Anspruch 11, dadurch gekennzeichnet, daß die Dichtmembran (435;535;635;735) mit Silikonöl einer Viskosität von 350 cSt bis 30.000 cSt silikoniert ist.

## Claims

1. A protector for a ventilating duct (21) of a liquid transfer device, particularly in a drip chamber (16), comprising a plug (426; 526; 626; 726) with an axial passage, which is adapted to be positioned at an opening of the ventilating duct (21) and is provided with a nonreturn valve comprising a flexible sealing diaphragm (435; 535; 635; 735) biased in shut-off direction at its inner side to be faced to the ventilating duct (21) and with a bacterial filter (440; 540; 640; 740) at its outer side to be faced away from the ventilating duct (21), the plug comprising an annular seat (434; 534; 634; 734) for the sealing diaphragm and an abutment (438; 538; 536; 738) for mounting the edge of the bacterial filter (440; 540; 640; 740) configured in disk-shape,
**characterized in**
that the sealing diaphragm (435; 535; 635; 735) is mounted adheringly and/or clampingly only over a portion of its outer circumference or comprises a circumferential adhering and/or clamping edge mounting, wherein, in the case of the circumferential edge mounting, the sealing diaphragm (735) is arranged, via a deflectable radial section (739), within a ring (735a) forming the edge mounting and surrounding it at a radial distance.

2. The protector according to claim 1, characterized in that the plug (426; 526; 626; 726) is inserted in a laterally directed pipe member (20) at the ventilating duct (21).

3. The protector according to claim 2, characterized in that the annular seat (534; 634; 734) is formed at the inner end of the plug (526; 626; 726).

4. The protector according to claim 2, characterized in that the annular seat (434) is formed at an end of an insert (445) which is configured as an axially pierced molded body fit into a central cavity (441) of the plug and whose other end carries an axial clamping element (448) for pressing the bacterial filter (440) against an abutment shoulder (438) of the plug (426).

5. The protector according to one of claims 1 to 4, characterized in that the annular seat (434; 734) has a triangular cross section.

6. The protector according to one of claims 1 to 3, characterized in that the annular seat (534; 634) extends obliquely towards the ventilating duct (21) under an angle of preferably 3° from the mounting of the sealing diaphragm (535; 635) to the plane crossing the longitudinal axis of the plug (526; 626) at right angles.

7. The protector according to one of claims 2 to 5, characterized in that the ring (735a) is clamped between an end face of an annular portion (729) of the plug (726) surrounding the seat (734) and a counter surface (730) at the bottom of the pipe member (20).

8. The protector according to claim 7, characterized in that the end face of the annular portion (729) and the counter surface (730) conically extend in a countersunk manner under an angle >0° and <90° to the plane crossing the longitudinal axis of the plug (726) at right angles.

9. The protector according to one of claims 1 to 8, characterized in that a curled edge (64; 664; 764) of the plug (526; 626; 726) presses the bacterial filter (540; 640; 740) against an abutment shoulder (538; 638; 738).

10. The protector according to one of claims 4 to 9, characterized in that the axial piercing of the plug (426; 526; 626; 726) and/or the insert (445) is formed by a concentric bore (442; 542; 642; 742).

11. The protector according to one of claims 1 to 10, characterized in that the sealing diaphragm (435; 535; 635; 735) is formed of natural rubber or thermoplastic elastomer material.

12. The protector according to claim 11, characterized in that the sealing diaphragm (435; 535; 635; 735) is silicone-treated with silicone fluid of a viscosity of from 350 cSt to 30,000 cSt.

## Revendications

1. Dispositif de protection pour un canal de prise d'air (21) d'un appareil de transfusion de liquide, notamment dans le cas d'une chambre compte-gouttes (16), constitué d'un bouchon (426; 526; 626; 726) à passage axial, qui peut être placé sur une ouverture du canal de prise d'air (21), et qui est pourvu sur sa face intérieure devant être dirigée vers le canal de prise d'air (21), d'un clapet anti-retour présentant une membrane d'étanchéité flexible (435; 535; 635; 735) précontrainte dans la direction de la fermeture, et sur sa face extérieure devant être dirigée à l'opposé du canal de prise d'air (21), d'un filtre anti-bactéries (440; 540; 640; 740), le bouchon présentant un siège annulaire (434; 534; 634; 734) pour la membrane d'étanchéité et un appui (438; 538; 638; 738) pour la fixation du bord du filtre anti-bactéries (440; 540; 640; 740) réalisé sous forme de disque, **caractérisé** en ce que la membrane d'étanchéité (435; 535; 635; 735) est fixée par adhérence et/ou par serrage uniquement sur une partie de sa périphérie extérieure, ou bien présente une fixation de bordure, périphérique, par adhérence et/ou par serrage, la membrane d'étanchéité (735) étant disposée, dans le cas de la fixation de bordure, périphérique, par l'intermédiaire d'un tronçon radial (739) pouvant être dévié, à l'intérieur d'un anneau (735a) formant la fixation de bordure et entourant la membrane d' étanchéité à distance radiale.

2. Dispositif de protection selon la revendication 1, **caractérisé** en ce que le bouchon (426; 526; 626; 726) est inséré dans un embout (20) orienté latéralement sur le canal de prise d'air (21).

3. Dispositif de protection selon la revendication 2, **caractérisé** en ce que le siège annulaire (534; 634; 734) est formé sur l'extrémité intérieure du bouchon (526; 626; 726).

4. Dispositif de protection selon la revendication 2, **caractérisé** en ce que le siège annulaire (434) est formé sur une extrémité d'un insert (445), qui est réalisé sous la forme d'un corps moulé transpercé axialement et ajusté dans une cavité centrale (441) du bouchon (426), et dont l'autre extrémité porte un organe de serrage axial (448) destiné à comprimer le filtre anti-bactéries (440) contre un épaulement d'appui (438) du bouchon (426).

5. Dispositif de protection selon l'une des revendications 1 à 4, **caractérisé** en ce que le siège annulaire (434; 734) présente une forme triangulaire en coupe transversale.

6. Dispositif de protection selon l'une des revendications 1 à 3, **caractérisé** en ce que le siège annulaire (534; 634) s'étend, à partir de la fixation de la membrane d'étanchéité (535; 635), de manière oblique par rapport au plan coupant perpendiculairement l'axe longitudinal du bouchon (526; 626), en formant un angle, de préférence de 3°, en direction du canal de prise d'air (21).

7. Dispositif de protection selon l'une des revendications 2 à 5, **caractérisé** en ce que l'anneau (735a) est serré entre une surface frontale d'une partie annulaire (729) du bouchon (726), entourant le siège (734), et une contre-surface (730) située au fond de l'embout (20).

8. Dispositif de protection selon la revendication 7, **caractérisé** en ce que la surface frontale de la partie annulaire (729) et la contre-surface (730) s'étendent sous une forme conique en creux par rapport au plan coupant perpendiculairement l'axe longitudinal du bouchon (726), en formant avec celui-ci un angle > 0° et < 90°.

9. Dispositif de protection selon l'une des revendications 1 à 8, **caractérisé** en ce qu'une collerette rabattue (64; 664; 764) du bouchon (526; 626; 726) comprime le filtre anti-bactéries (540; 640; 740) contre un épaulement d'appui (538; 638; 738).

10. Dispositif de protection selon l'une des revendications 4 à 9, **caractérisé** en ce que le passage axial du bouchon (426; 526; 626; 726) et/ou de l'insert (445) est formé par un alésage concentrique (442; 542; 642; 742).

11. Dispositif de protection selon l'une des revendications 1 à 10, **caractérisé** en ce que la membrane d'étanchéité (435; 535; 635; 735) est réalisée en caoutchouc naturel ou en un matériau élastomère thermoplastique.

12. Dispositif de protection selon la revendication 11, **caractérisé** en ce que la membrane d'étanchéité (435; 535; 635; 735) est siliconée, à l'aide d'une huile aux silicones d'une viscosité de 350 cSt à 30.000 cSt.
